(19) Europäisches Patentamt
European Patent Office
Office européen des brevets

(11) Publication number: 0 325 825 B1

(12) EUROPEAN PATENT SPECIFICATION

(45) Date of publication of patent specification: 16.09.92 (51) Int. Cl.5: C09K 11/06, C09D 5/22

(21) Application number: 88300743.7

(22) Date of filing: 28.01.88

(54) **Phosphorescent materials.**

(43) Date of publication of application:
02.08.89 Bulletin 89/31

(45) Publication of the grant of the patent:
16.09.92 Bulletin 92/38

(84) Designated Contracting States:
AT BE CH DE ES FR IT LI NL SE

(56) References cited:
EP-A- 0 130 117
GB-A- 1 146 522
GB-A- 1 494 103
GB-A- 2 016 370
GB-A- 2 194 244

(73) Proprietor: THE POST OFFICE
Postal Headquarters 33 Grosvenor Place
London SW1X 1PX(GB)

(72) Inventor: Powell, David Cyril
33 Green Way
Tunbridge Wells Kent(GB)
Inventor: Walker, Aubrey Douglas
157 Northumberland Road
North Harrow Middlx(GB)

(74) Representative: Silverman, Warren et al
HASELTINE LAKE & CO. Hazlitt House 28
Southampton Buildings Chancery Lane
London WC2A 1AT(GB)

EP 0 325 825 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND TO THE INVENTION

This invention relates to organic phosphors comprising molecules of a phosphorescence activator in a matrix of a condensation resin that does not itself absorb ultra-violet or visible radiation at the excitation and emission frequencies of the phosphorescence activator, which phosphorescence activator utilises singlet-triplet transitions.

Phosphors of such type are known for example from British Patent Specifications Nos. 870,504, 1,494,102 and 1,493,103. Such phosphors may contain as a matrix a cross-linked condensation resin, the resin being formed by condensation in the presence of phosphorescence activators so that the molecules of the latter are trapped and isolated from one another in the matrix. The resin is preferably a condensation product of formaldehyde, generally with an amine group-containing compound, especially urea or melamine. The phosphor is a solid, generally insoluble in water and organic solvents, and, for use as a phosphorescent printing ink, may be ground finely and dispersed in a suitable solvent, for example toluene.

The phosphorescence activators which have hitherto been proposed fall within a variety of classes of organic compounds. Examples of such compounds include carbazole sulphonic acid, naphthalene disulphonic acid, diphenyl guanidine and 2-aminobenzoflavone. However the mean intensity of the phosphorescent emission obtained with such compounds is often mediocre for practical use in for example security marking of documents and products.

Phosphorescence, as a phenomenon, is to be distinguished from other light-generating phenomena to which the term luminescence may be applied, which term is generic, too, to phosphorescence. The most common such phenomenon is fluorescence which, in contrast to phosphorescence, is the result of triplet-triplet transitions. EP-A-130117 discloses luminescent materials manifesting fluorescence and comprising an organic compound, e.g.

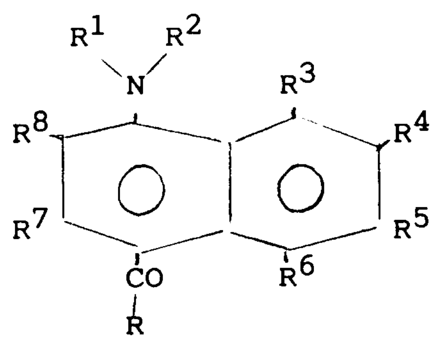

wherein R may be an alkyl radical, $R^1$ and $R^2$ are alkyl and $R^3$ to $R^8$ are hydrogen, alkyl, alkoxy or halogen. This compound is disposed in a rigid matrix which can be a polymer, a glass or a hydrated salt and which may or may not be transparent.

SUMMARY OF THE INVENTION

It is an object of this invention to provide organic phosphors showing improved phosphorescent behaviour.

According to the present invention there are provided organic phosphors as aforesaid wherein there is present a phosphorescence activator which is a compound of the general formula

$(A)_m$ - Ar - CO - X (I)

wherein Ar denotes a fused ring polycyclic aromatic group;

m is zero or an integer of from 1 up to the maximum number of ring positions in Ar available for substitution;

X denotes an alkyl group or a group of the formula $(A)_m$-Ar- or (B)n$\phi$- wherein $\phi$ is a phenyl group and n is zero or an integer of from 1 to 5; and

A and B represent ring substituents;

or an acid addition salt thereof when said compound is basic.

In the aforesaid general formula, Ar is preferably an optionally substituted hydrocarbyl group, especially naphthyl, anthryl, phenanthryl, acenaphthyl or acenaphthenyl group. However, it is also possible for a heterocyclic ring to be fused to an aromatic ring which is bonded to the acyl group X-CO- as in a carbazolyl group.

The substituents A and B, if present, may be any one of the following radicals:

alkyl or cycloalkyl - R
phenoxy - O $\phi$ or alkoxy or cycloalkoxy - OR
hydroxy - OH
phenyl - $\phi$
amino - $NH_2$
amido

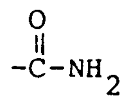

carboxyl

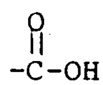

(or salt or ester)
sulphonic acid $-SO_3H$ (or salt or ester)
halogeno - F, - Cl, - Br, - I
cyano - CN
azido - $N_3$ or optionally substituted phenylazo
i.e. B - $\phi$ - N = N
hydrazino - $NHNH_2$
aldehyde - CHO

The group A or B may also be a combination of the aforementioned radicals as for example in a tertiary amino group which may have the formula - $NR_3$, a halogenated alkyl group such as $-CF_3$ or a substituted phenyl group.

The compounds of the formula I form a valuable class of substances which are capable of displaying phosphorescence under suitable conditions when incorporated in organic phosphors as aforesaid, in which they are preferably present in an amount of from 0.25 to 10 per cent by weight, based on the total weight of resin and activator.

The aforementioned compounds may be prepared by Friedel Crafts acylation of a polycyclic aromatic hydrocarbon or substituted derivative thereof with the appropriate acid chloride or acid anhydride. The essential reaction is as follows:

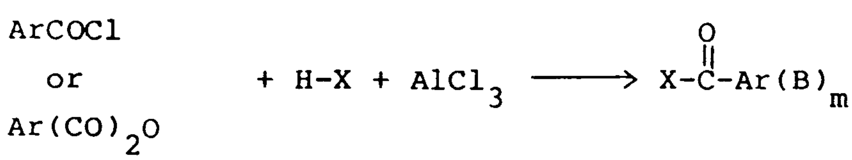

This method is both simple and suitable for commercial development despite the disadvantage of the possibility of forming more than one ketonic substituted isomer. The isomers may be isolated in pure form or for reasons which will be apparent from the foregoing, may be used in admixture in the aforementioned phosphor compositions. In general, herein, where reference is made to the structure of a specific isomer unless otherwise indicated, this will indicate the major isomer predicted for the Friedel Crafts reaction utilised in a specific comparative procedure. In general, limited purification of the product has been carried out followed by chromatographic analysis and the product obtained (found in most cases to be 90% pure) was utilised in subsequent tests.

In practice, the selection of starting reactants will depend upon the reaction conditions to be employed

and the use of the desired product. Thus having regard to the Friedel Crafts reaction, it has been found that naphthalene and phenanthrene give good results. It would also be expected that anthracene, being another simple fused hydrocarbon would give similar results. Having regard to the intended use of the phosphors including the compounds in printing inks, substitution of the groups Ar and X may be provided to render the compounds of formula I soluble in the solvent of the ink. This may be provided by substitution in the starting compounds or by substitution of the product of the Friedel Crafts reaction. In the latter case, it is possible that there may be produced a mixture of compounds depending upon the course of substitution and again, it may be more convenient to utilise such mixtures of compounds in the aforementioned phosphor compositions. A particularly preferred type of substitution in this respect is through sulphonation of the Friedel Crafts reaction product.

According to one embodiment of the invention, there is proposed the use of a compound or mixture of compounds of the formula II;

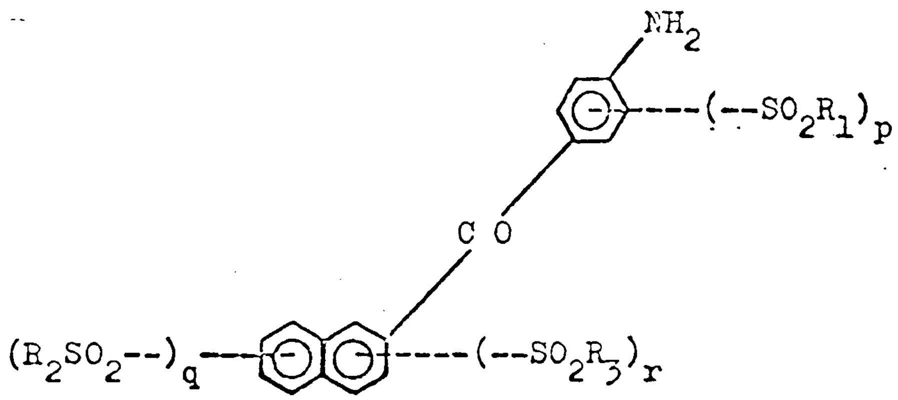

(II)

wherein:

each of $R_1$, $R_2$ and $R_3$, which may be the same or different, represents

-OH,

-OM where M represents a monovalent cation or l/m of an m-valent cation,

a halogen atom, or

-OR where R represents an unsubstituted or substituted alkyl, aryl or cycloalkyl radial,

p represents O or an integer from 1 to 4,

q represents O or an integar from 1 to 4,

r represents O or an integar from 1 to 3.

The unsulphonated compounds of the formula Ia:

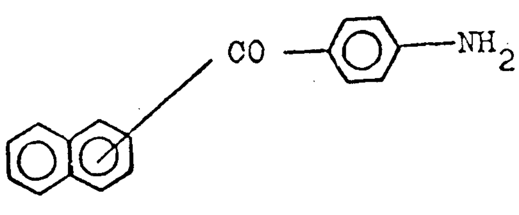

(IIà)

and the free sulphonic acids and their salts of the formula IIb

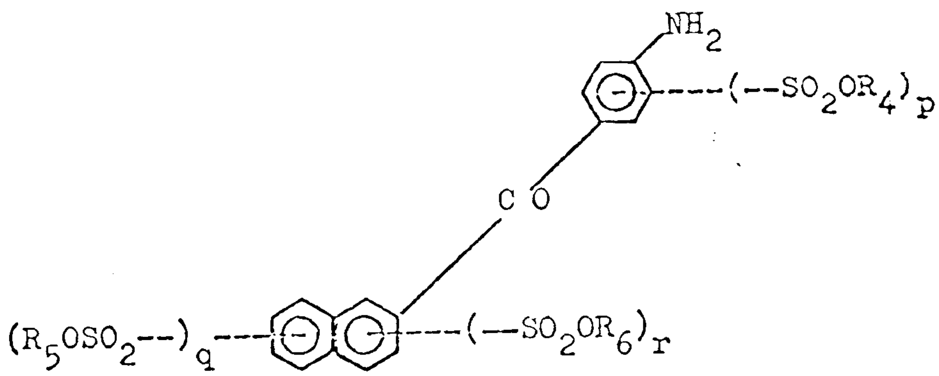

(IIb)

wherein each of $R_4$, $R_5$ and $R_6$, which may be the same or different, represents H or M, where M is as previously defined, are of especial interest. Phosphors incorporating these compounds and mixtures thereof are capable of displaying high-intensity phosphorescence under suitable conditions, producing a brilliant yellow-green afterglow.

The production of compounds used in phosphors embodying this invention will not be described in greater detail with reference to the compounds of formulae (II), (IIa) and (IIb), but it should be appreciated that analogous reaction procedures may be employed to produce any compound of the present invention in accordance with the nature of radicals Ar, X and A.

Insofar as the Friedel Crafts reaction for acylating the nucleus of a polycyclic aromatic compound is concerned, particularly in producing compounds of general formula IIa, this may be carried out by the Friedel-Crafts acylation of naphthalene with a p-nitrobenzoyl halide, for example, the chloride, followed by reduction of the nitro group to an amino group. The first stage may be carried out using an aluminum chloride catalyst in an inert solvent such as carbon disulphide, and the second, reduction, stage may be effected, for example, by means of stannous chloride in the presence of hydrochloric acid; the reaction scheme may be represented as follows:

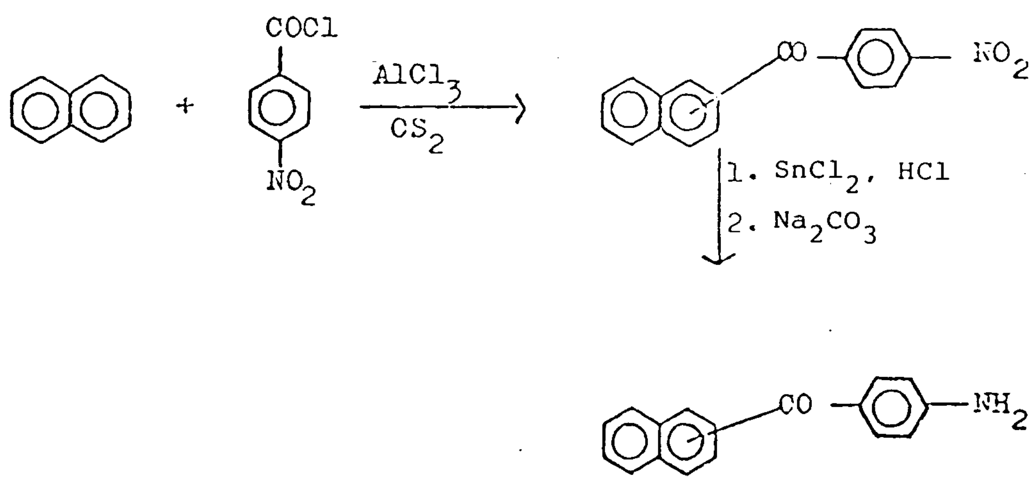


(III)

(IIa)

The first stage of this reaction scheme gives a mixture of α- and β-substituted products of the formula III, the proportions depending on the temperature and duration of the reaction. The second stage is indicative of reactions which may be carried out to modify substituents already present in the product of the Friedel Crafts reaction. The second stage does not alter the distribution of the isomers. The subsequent sulphonation reaction yields a mixture of products of different degrees of sulphonation. The number and position of the sulphonic acid groups or of sulphonic acid derivative groups does not appear to be relevant to the phosphorescence property and the mixture of products obtained by the sulphonation process may be used as a phosphor without there being any necessity for separation. It is believed that the mixed sulphonated product has on average 2 to 3 sulphonic acid groups or sulphonic acid derivative groups per molecule.

This procedure, with the exclusion of a reduction reaction using stannous chloride, that is a reaction scheme wherein a Friedel Crafts reaction is followed by sulphonation, has also been utilised to produce

sulphonated benzoyl naphthalene of formula:

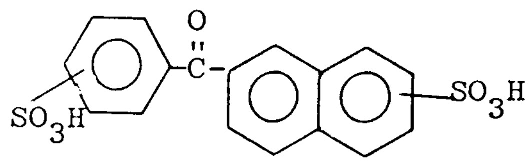

and also sulphonated naphthoylnapthalene of the formula:-

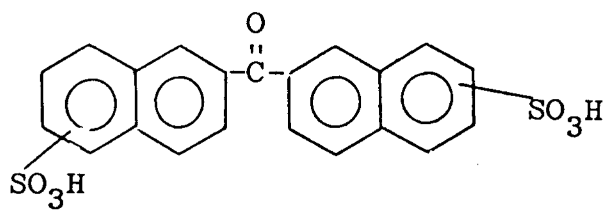

However as will be apparent from the foregoing, the present invention is not limited to the sulphonated compounds and other compounds having the character of phosphorescence activators for use in organic phosphors according to the invention which have been produced are the following:

3-(4-aminobenzoyl)phenanthrene
3-(4-methoxybenzoyl)phenanthrene
3-benzoylphenanthrene
3-acetylphenanthrene
3-(2-carboxybenzoyl)phenanthrene
3-(1-carboxynaphth-8-oyl)phenanthrene
3-(4-aminobenzoyl)carbazole
2-(4-aminobenzoyl)naphthalene
2-(4-aminobenzoyl)anthracene
3-(4-aminobenzoyl)acenaphthylene
2-(4-methoxybenzoyl)naphthalene
2-(4-hydroxybenzoyl)naphthalene
2-(4-carboxybenzoyl)naphthalene
2-(2-carboxybenzoyl)naphthalene
2-(4-chlorobenzoyl)naphthalene
2-(4-n-butylbenzoyl)naphthalene
2-(4-azidobenzoyl)naphthalene
2-(4-hydrazinobenzoyl)naphthalene
2-[4-(4-hydroxyphenylazo)-benzoyl]naphthalene
2-(naphth-2-oyl)-naphthalene
2-(naphth-2-oyl)phenanthrene

Phosphors of the present invention will, under suitable conditions, exhibit intense phosphorescence during and after irradiation with ultra violet light. In practice either long-wavelength UV (365 nm) or short-wavelength UV (254 nm) is generally used to induce phosphorescence, and the phosphors of the invention show a good response to both. The phosphorescence represents the radiative decay of a triplet excited state to the singlet ground state; this transition is forbidden and the triplet state has a relatively long lifetime, so that afterglow occurs. Collision-induced non-radiative decay pathways are more favourable and will always predominate under conditions in which molecular interaction is possible, for example, in the liquid phase. To obtain observable phosphorescence it is necessary to prevent non-radiative decay by isolating the molecules from one another in a rigid matrix. The matrix itself must, of course, be reasonably transparent to radiation at the absorption and emission frequencies of the phoshporescent molecule, which is generally referred to in this context as the phosphorescence activator.

The organic phosphors of the present invention will generally be produced by methods known per se but utilising the phosphorescence activators which, according to the present invention, have been found to be particularly suitable for use therein. Thus, one form of matrix which may be used successfully in connection with prior art phosphorescence activators, and is claimed, in British Patent Specifications

870,504, 1,494, 102 and 1,494,103.

The resin is preferably a condensation product of formaldehyde, more preferably with an amino compound, especially urea or melamine, and is advantageously as described in any of the three British patent specifications mentioned above.

A phosphor embodying this invention is a solid insoluble in water and organic solvents, and for use as a phosphorescent printing ink may be ground finely and dispersed in a suitable solvent, for example, toluene.

The phosphor may, for example, be in the form of printed matter on a substrate, especially paper or plastics material, or in the form of a coating on paper. As a further possibility encompassed by the invention, the condensation resin of such a phosphor may have been formed in situ on the substrate. An ink or coating of this type is described and claimed in British Patent Specification No.1,494,102, and the in situ condensation process is carried out as described and claimed in British Patent Specification No.1 494 103. Such an ink or coating which acts as a precursor for the organic phosphor of this invention comprises, in an aqueous solution, at least one phosphorescence activator which is a compound of such type essentially for use in the present invention and which is water soluble and further comprises as solute a soluble precondensate obtained by a reaction between two or more components, the soluble precondensate being capable of further reaction in the absence of the phosphorescence activator(s) to form an insoluble condensation product that does not substantially absorb ultraviolet radiation at any wavelength at which the phosphorescence activarot(s) substantially absorb ultraviolet radiation and which is capable of such further reaction in the presence of the phosphorescence activator(s) to form a phosphorescent insoluble condensation product.

An especially advantageous effect may be obtained if the phosphorescence activator of the invention is used in conjunction with another activator the emission of which is of different wavelength and of different duration. The observed afterglow then appears to change colour with time; moreover the presence of two different emission frequencies may be detected with suitable apparatus and the response of the phosphor to long-wavelength (365 nm) UV and short-wavelengths (254 nm) UV may also be different, if one of the two activators is activated only by short-wavelength UV.

One activator, already known per se, which is especially suitable for use in conjunction with the activator of the present invention is carbazole sulphonic acid, either as the free acid or in the form of a salt for example, the sodium salt. This activator produces a blue afterglow of a longer duration than that of the afterglow of the activator of the invention. The afterglow of a phosphor containing both activators thus changes from yellow-green to blue over a period of a few seconds. The weight ratio of the phosphorescence activator used essentially according to this invention to such a carbazole sulphonic acid is preferably within the range 0.2 to 1.0, based on the sodium salts of the two activators.

The following Example illustrate the invention.

Example 1

Preparation of p-aminobenzoyl naphthalene (formula IIa)

Naphthalene (1500g) and p-nitrobenzoyl chloride (1800 g) were dissolved in carbon disulphide (8 litres) in a 20-litre flask. The flask was fitted with a mercury sealed stirrer, reflux condenser, and from the reflux condenser an HCl gas absorption device.

Anhydrous aluminium powder (1400 g) was added in small portions with stirring, over 2 hours. HCl was evolved and a dark brown granular product separated. The reactants were allowed to stand for 24 hours and the product filtered off. The dark brown product was washed with petroleum spirit (40/60) (about 2 litres) and air-dried. It was then added cautiously to crushed ice (5 kg), concentrated HCl (2 litres) and water (10 litres) until hydrolysis of the aluminium chloride complex was complete. The product was filtered off and air-dried.

Stannous chloride (7 kg) was dissolved, with warming, in concentrated HCl (8 litres) and the product from above added, with warming, in small portions. Since the reaction is strongly exothermic, the product was added at such a rate as to control the reaction (boiling gently). The mixture was then left to stand for 24 hours and a brown solid separated which was filtered off and air-dried.

The product, as the stannic chloride complex, was dissolved in a minimum volume of warm acetone. Solid anhydrous sodium carbonate was then added until effervescence ceased (water being added to speed up the reaction). The dark acetone solution was filtered through kieselguhr, and a small volume of water added until precipitation commenced.

The filtrate was then boiled for 10 mins with decolourising charcoal. The charcoal was filtered off and the product precipitated, as a dark red oil, with excess water. When the oil had solidified, it was

recrystallised from a minimum volume of boiling methanol (this solution was also decolourised with charcoal). The yellow product which crystallised from methanol was filtered off and thoroughly dried; the yield was about 1 kg. This product was suitable for use as a phosphorescence activator without further purification.

Example 2

Sulphonation of p-aminobenzoyl naphthalene

500 g of the thoroughly dried p-aminobenzoyl naphthalene obtained in Example 1 were powdered and placed in a 5-litre flask. 500 ml of oleum (20% free $SO_3$) were added very cautiously, stirring with a glass rod. The rate of addition of oleum was such that a mobile red syrup was produced with very few fumes. The syrup was then added cautiously to water (about 3 litres). Most of the syrup was water-soluble; a small amount of insoluble by-product was filtered off and discarded.

The aqueous solution of sulphuric acid was then neutralised with NaOH solution. The resulting aqueous liquors were evaporated under reduced pressure to a volume of about 2 litres, cooled and a mass of $Na_2SO_4$ by-product crystallised out which was filtered off. The solution was evaporated further and more $Na_2SO_4$ removed.

The deep brown solution was finally evaporated to dryness, further dried in an oven and ground up. The organic content was determined by combustion, and the product was found to consist of about 60 per cent by weight of p-aminobenzoyl naphthalene sodium sulphonates, the remainder being sodium sulphate. This product was suitable for use as a phosphorescence activator without further purification.

Example 3

Preparation of a particulate organic phosphor

(a) Urea (5 kg) was melted and heated until it boiled gently. 40 g of the product obtained in Example 2 were added and heating was continued until the molten urea began to be turbid. During the heating stage, decomposition of the urea into various products, chiefly cyanuric acid, was occurring; the onset of turbidity indicated that the solubility of cyanuric acid in urea had been exceeded, and the heating was discontinued at this point.

Paraformaldehyde (1950 g) was added gradually to the reaction mixture with continuous stirring, slight heat being applied when necessary to keep the mixture molten. The fully mixed product was heat-cured at 150 ° C for 2 to 4 hours, allowed to cool, and then ground to a fine powder.

(b) The procedure just described was then repeated using the unsulphonated product of Example 1.

Example 4

Preparation of a particulate two-component organic phosphor

A particulate organic phosphor containing, as phosphorescence activator, the sodium salt of carbazole sulphonic acid was prepared by a method analogous to that described in Example 3(a), the 40 g of the product of Example 2 being replaced by 165 g of the sodium salt of carbazole sulphonic acid. After grinding, 1 part by weight of the finely powdered product was thoroughly mixed with 2 parts by weight of the product of Example 3(a).

Example 5

Preparation of a particulate two-component organic phosphor - alternative method

Instead of a physical mixture of two separately prepared phosphors, the two-component phosphor may be a composite product made by carrying out the urea-formaldehyde condensation in the presence of a mixture of the two activators. In this procedure, the methods described in Example 3(a) may again be used, with a mixture of 27 g of the product of Example 2 and 67 g of the sodium salt of carbazole sulphonic acid replacing the 40 g of the former product used in Example 3(a).

Example 6

Preparation of an aqueous solution phosphorescent printing ink

An aqueous solution phosphorescent ink was prepared by the method described in Example 1 of British Patent Specification No.1,494,102, using 8 g of the product of Example 2 above instead of 20 g of carbazole sulphonic acid sodium salt.

A two-component ink may be produced either by mixing two single-activator inks prepared in this manner, or by carrying out the preparation described above using 5.3 g of the product of Example 2 and 13 g of sodium carbazole-sulphonate.

## Example 7

Preparation of a paper-coating mix

A paper-coating mix may be prepared by any of the methods described in Examples 2 to 4 of British Patent Specification No. 1,491,102, if the 24 g of p-aminobenzoic acid used in those Examples are replaced either by 10 g of the free acid of the product of Example 2 or by a mixture of 16.6 g of carbazole sulphonic acid and 6.6. g of the free acid of the product of Example 2.

Similarly, the method of Example 5 of that specification may be used if the 30 g of p-aminobenzoic acid are replaced by either 12 f of the free acid of the product of Example 2 or by a mixture of 20 g of carbazole sulphonic acid and 8 g of the free acid of the product of Example 2.

## Example 8

Comparison of phosphorescence intensities

A series of phosphors was prepared by the method described in Example 3 but using the various activators numbered 1, 2 and 4 to 18 in the Table that follows. Samples of these phosphors, and a sample of the phosphor prepared in Example 3(a) (numbered 3 in the Table), were prepared in the form of 1/1000 inch ($2.54 \times 10^{-5}$ m) drawdowns, and were irradiated with a long-wavelength ultraviolet light (365 nm), for a period of 200 ms. The mean intensity of the phosphorescent emission for each specimen was recorded under longwave UV excitation (365 nm) using a Post Office designed phosphorescence-measuring apparatus fitted with a blue-sensitive photo multiplier detector after a delay period of 150 ms. The relative after glow intensities (in arbitary units) are shown in the following Table I. It is clear that the intensity of emission of sulphonated p-aminobenzoyl naphthalene after long-wavelength irradiation is considerably higher than that of any of the other activators tested.

Table I

| Phosphorescence Activator | Mean Brightness |
|---|---|
| 1. Carbazole sulphonic acid | 48 |
| 2. p-aminobenzophenone | 65 |
| 3. Sulphonated p-aminobenzoyl naphthalene | 231 |
| 4. p-hydroxy diphenyl sulphonic acid | 49 |
| 5. Fluorene sulphonic acid | 3 |
| 6. Diphenylene oxide sulphonic acid | 5 |
| 7. 1-Naphthylamine 2-sulphonic acid | 29 |
| 8. $\overline{2}$-Naphthylamine 5, 9 Disulphonic acid (amino-J-acid) | 23 |
| 9. $\overline{\alpha}$-Naphthoflavone | 100 |
| 10. Vanillic acid (4-Hydroxy 3-methoxy benzoic acid) | 12 |
| 11. 3-Hydroxy diphenylamine sulphonic acid | 25 |
| 12. Diphenylamine | 2 |
| 13. Diphenylguanidine | 17 |
| 14. Acridone | 61 |
| 15. 2-Aminobenzoflavone | 39 |
| 16. Methyl umbelliferone | 40 |
| 17. 2-Naphthylamine 6, 8 Disulphonic acid (amino G-acid) | 12 |
| 18. 2-Naphthol $\epsilon$-sulphonic acid (Schaeffer's acid) | 7 |

EXAMPLE 9

A series of further compounds was prepared by a Friedel Crafts method from a polycyclic substrate compound and a corresponding acylation agent by the aforementioned Friedel Crafts method. Limited purification of the product obtained was carried out followed by chromatographic analysis and the product obtained (in most cases 90% pure) was incorporated in a urea formaldehyde pot melt in a concentration of 1% by weight. In each case, the phosphorescence colour was determined. The results obtained are set out in the foregoing Table II:

T a b l e II

Table of Compounds Prepared

| Compound | Structure | Chromatography* | Phosphorescence in U/F Hot Melt |
|---|---|---|---|
| (1) 3-(4-amino benzoyl) phenanthrene | 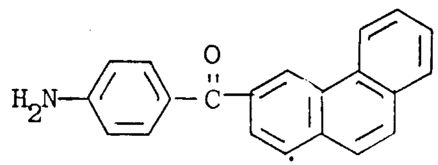 | main spot >90% | green very bright |
| (2) 3-(4-methoxy benzoyl) phenanthrene | 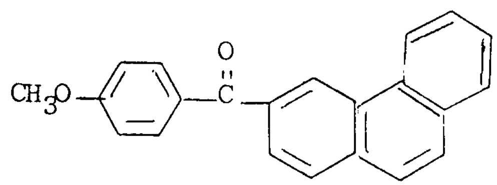 | main spot >90% | green very bright |
| (3) 3-Benzoyl phenanthrene | 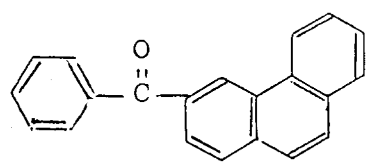 | main spot >90% | green bright |
| (4) 3-Acetyl phenanthrene | 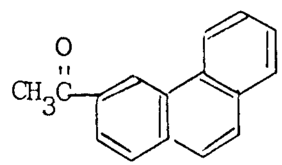 | main spot >90% | green not as bright as (3) |

| | | | |
|---|---|---|---|
| (5) 3-(2-Carboxy benzoyl) phenanthrene | 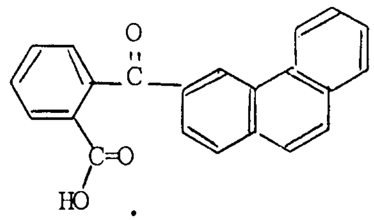 | main spot > 90% | green very bright |
| (6) 3-(1-Carboxy naphth-8-oyl) phenanthrene | 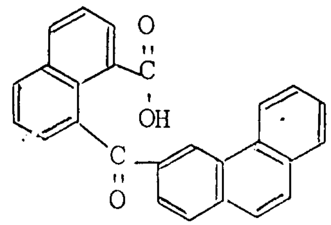 | mixture of products main spot ~ 50% of total | orange poor |
| (7) 3-(4-amino benzoyl) carbazole | 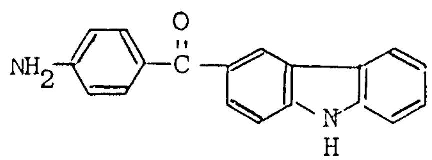 | unsuccessful separation in this system | blue green poor |

***Chromatography:-** TLC on silica gel using n-Hexane : Acetone 4:1 developer, visualised by fluorescence and UV absorption.

In general, it appears that 3-benzoyl phenanthrenes substituted in the 2 and 4 positions in the benzoyl group amino-methoxy or carboxylic acid groups give rise to very bright activators. Compounds 1 to 4 are insoluble in aqueous solvents and would need to be sulphonated before use in aqueous inks. Sulphonation of compound 2 would have been difficult because of the lability of the methoxy group under strongly acidic conditions.

Experiments have shown that compound (5) is however the most satisfactory compound overall for the following reasons:

A. It has excellent characteristics as a phosphor activator.

B. It is soluble in aqueous solvents in the form of its sodium salt.

C. It does not contain an amino group attached to an aromatic hydrocarbon, thereby being acceptable on health and safety grounds.

D. It can be prepared at relatively low cost from low cost starting materials.

## Claims

**1.** An organic phosphor comprising molecules of a phosphorescence activator in a matrix of a condensation resin that does not itself absorb ultra-violet or visible radiation at the excitation and emission frequencies of the phosphorescence activator which phosphorescence activator utilises singlet-triplet transitions and is a compound of the general formula

$(A)_m$ - Ar - CO - X (I)

wherein Ar denotes a fused ring polycyclic aromatic group;

m is zero or an integer of from 1 up to the maximum number of ring positions in Ar available for substitution;

X denotes an alkyl group or a group of the formula $(A)_m$-Ar- or $(B)_n\phi$- wherein $\phi$ is a phenyl group and n is zero or an integer of from 1 to 5; and

A and B represent ring substituents;

or an acid addition salt thereof when said compound is basic.

**2.** A phosphor as claimed in claim 1, wherein Ar is selected from naphthyl, anthryl, phenanthryl, acenaphthyl and acenaphthenyl groups.

**3.** A phosphor as claimed in claim 1 or 2, wherein A and B represent ring substituents selected from alkyl, cycloalkyl, phenyl, alkoxy, cycloalkoxy, phenoxy, hydroxy, primary amino, hydrazino, amido, carboxyl, aldehyde, sulphonic acid, halogeno, cyano, azido and phenyl azo and substituent derivatives thereof.

**4.** A phosphor as claimed in claim 1, which contains, as a phosphorescence activator 2-(4-aminobenzoyl) naphthalene.

**5.** A phosphor as claimed in claim 1, which contains as a phosphorescence activator 3-(4-aminobenzoyl) phenanthrene.

**6.** A phosphor as claimed in claim 1, which contains as a phosphorescence activator 2-(naphth-2-oyl)-naphthalene.

**7.** A phosphor as claimed in claim 1, which contains as a phosphorescence activator 3-(4-aminobenzoyl)-acenaphthylene.

**8.** A phosphor as claimed in claim 1, wherein the phosphorescence activator is a compound or mixture of compounds of the formula:

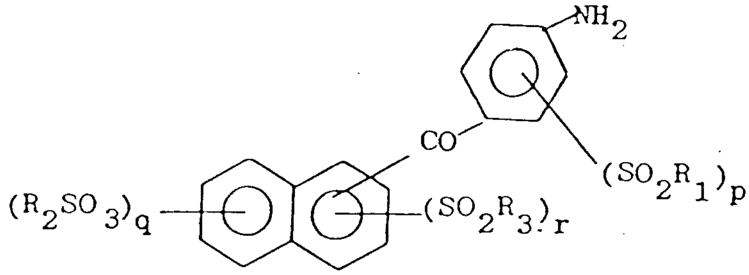

II

wherein:

each of $R_1$, $R_2$ and $R_3$ which may be the same or different represents

-OH

-OM wherein M represents a monovalent cation or $^1/m$ of an m-valent cation, a halogen atom, or

-OR wherein R represents an unsubstituted or substituted alkyl, aryl or cycloalkyl radical,

p represents zero or an integer from 1 to 4,

q represents zero or an integer from 1 to 4 and

r represents zero or an integer from 1 to 3 with the total of p + q + r being at least 1.

**9.** A phosphor as claimed in claim 8, wherein the phosphorescence activator is a compound or mixture of compounds having the formula

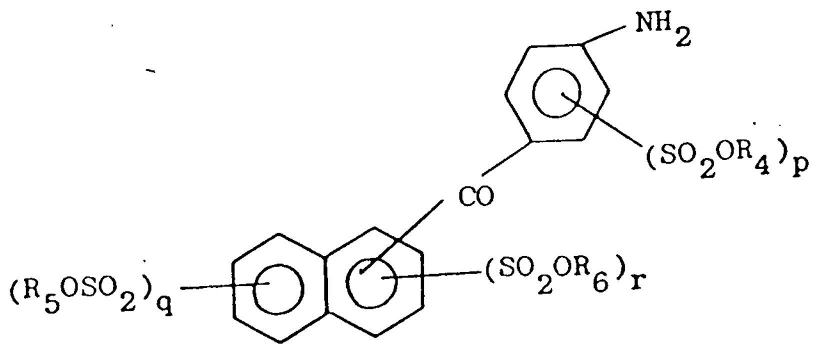

(IIb)

wherein each of $R_4$, $R_5$ and $R_6$ which may be the same or different represents H or M, wherein M is as defined in claim 9.

**10.** A phosphor as claimed in claim 8 or 9, wherein the total of p + q + r is 2.

**11.** A phosphor as claimed in claim 8 or 9, wherein the total of p + q + r is 3.

**12.** A phosphor as claimed in claim 8 or 9, wherein M represents an alkali metal cation.

**13.** A phosphor as claimed in any one of the preceding claims which includes a further phosphorescence activator which is carbazole sulphonic acid or a salt thereof.

**14.** A phosphor as claimed in claim 13, wherein the phosphorescence activator as defined in claim 1 and the carbazole sulphonic acid are present in a weight ratio within the range of from 0.2 to 1.0, based on the sodium salts of both activators.

**15.** A phosphor as claimed in any preceding claim wherein the matrix comprises a resin which is a condensation product of formaldehyde with an amine group-containing compound.

**16.** A phosphor as claimed in claim 15, wherein the amine group-containing compound is urea or melamine.

**17.** A phosphor as claimed in claim 15 or 16, which contains a total of from 0.25 to 10 per cent by weight of phosphorescence activator as defined in claim 1, based on the total weight of the resin and the activator.

**18.** A printing ink comprising a suspension of an organic phosphor as claimed in any one of the preceding claims in a suitable medium.

**19.** A precursor of an organic phosphor, which comprises an aqueous solution comprising as solute at least one phosphorescence activator, which is a compound as defined in any one of claims 1 to 12, which is water soluble, and further comprising as solute a soluble precondensate obtained by a reaction between two or more components, the soluble precondensate being capable of further reaction in the

absence of the phosphorescence activator to form an insoluble condensation product that does not substantially absorb ultraviolet radiation at any wavelength at which the phosphorescence activator substantially absorbs ultraviolet radiation and being capable of such further reaction in the presence of the phosphorescence activator to form a phosphorescent insoluble condensation product.

**20.** A phosphor precursor as claimed in claim 19, which includes as a further phosphorescence activator, carbazole sulphonic acid or a salt thereof.

**21.** A phosphor precursor as claimed in claim 20, wherein the weight ratio of activator as defined in claim 1 to carbazole sulphonic acid is within the range of from 0.2 to 1.0 based on the sodium salts of both activators.

**22.** A printing ink consisting essentially of a phosphor precursor as claimed in any one of claims 19 to 21.

**23.** A paper-coating composition comprising a phosphor precursor according to any one of claims 19 to 22 in admixture or conjunction with one or more fillers and/or binders and/or pigments and/or stabilisers and/or other additives.

**Patentansprüche**

**1.** Organischer phosphoreszenzfähiger Stoff, enthaltend Moleküle von einem phosphoreszierenden Aktivator in einer Matrix aus einem Kondensationsharz, das Ultraviolette und sichtbare Strahlung bei der Anregungs- und Emissions-Frequenz von dem phosphoreszierenden Aktivator selbst nicht absorbiert, wobei der phosphoreszierende Aktivator Singulett-Triplett-Umwandlungen ausnutzt und eine Verbindung gemäß der allgemeinen Formel

$(A)_m$ - Ar - CO - X (1)

in der
Ar eine kondensierte polycyclische aromatische Ringgruppierung bedeutet,
m 0 oder eine Zahl von 1 bis zur maximalen Anzahl der in Ar für eine Substitution zur Verfügung stehenden Rinpositionen darstellt,
X eine Alkylgruppe oder eine Gruppe entsprechend der Formel $(A)_m$ - Ar - oder $(B)_n\phi$- ist, wobei $\phi$ eine Phenylgruppe bedeutet und n 0 oder einer Zahl von 1 bis 5 entspricht sowie A und B Ringsubstituenten wiedergeben,
oder ein Säureadditionssalz ist, sofern es sich bei der Verbund um eine Base handelt.

**2.** Phosphoreszenzfähiger Stoff gemäß Anspruch 1, worin Ar ausgewählt ist aus Naphthyl-, Anthryl-, Pheanthryl-Acenaphthyl- und Acenaphthenyl-Gruppen.

**3.** Phosphoreszenzfähiger Stoff gemäß Anspruch 1 oder 2, wobei A und B Ringsubstituenten darstellen, die aus Alkyl, Cycloalkyl, Phenyl, Alkoxy, Cycloalkoxy, Phenoxy, Hydroxy, primäres Amin, Hydrazino, Amido, Carboxyl, Aldehyd, Sulfonsäure, Halogeno, Cyano, Azido und Pheylazo und substituierten Derivaten davon ausgewählt sind.

**4.** Phosphoreszenzfähiger Stoff gemäß Anspruch 1, enthaltend als phosphoreszierenden Aktivator 2-(4-Aminobenzoyl)naphthalin.

**5.** Phosphoreszenzfähiger Stoff gemäß Anspruch 1, enthaltend als phosphoreszierenden Aktivator 3-(4-Aminobenzoyl)phenanthren.

**6.** Phosphoreszenzfähiger Stoff gemäß Anspruch 1, enthaltend als phosphoreszierenden Aktivator 2-(Naphth-2-oyl)naphthalin.

**7.** Phosphoreszenzfähiger Stoff gemäß Anspruch 1, enthaltend als phosphoreszierenden Aktivator 3-(4-Aminobenzoyl)acenaphthylen.

**8.** Phosphoreszenzfähiger Stoff gemäß Anspruch 1, wobei der phosphoreszierende Aktivator eine Verbindung oder eine Mischung von Verbindungen entsprechend der Formel

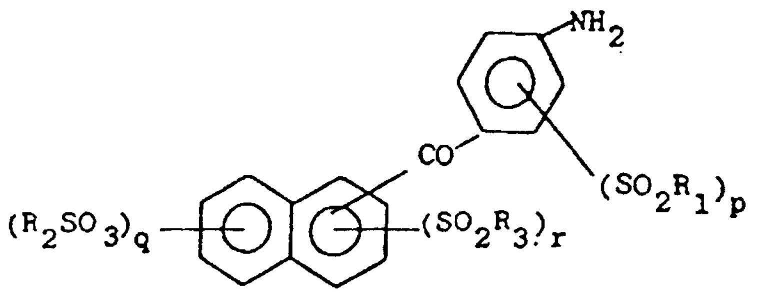

II

ist, worin
jeder der Substituenten $R_1$, $R_2$ und $R_3$, die gleich oder verschieden sein können, entsprechen:
-OH,
-OM mit M einem monovalenten Kation oder 1/m eines mehrwertigen Kations oder einem Halogenatom,
-OR mit R in der Bedeutung von unsubstituiertem oder substituiertem Alkyl-, Aryl- oder Cycloalkylrest,
p 0 oder eine ganze Zahl von 1 bis 4 ist,
q 0 oder eine ganze Zahl von 1 bis 4 ist und
r 0 oder eine ganze Zahl von 1 bis 3 ist sowie die
Ganzheit von p + q + r wenigstens 1 entspricht.

**9.** Phosphoreszenzfähiger Stoff gemäß Anspruch 8, worin der phosphoreszierende Aktivator eine Verbindung oder eine Mischung von Verbindungen entsprechend der Formel

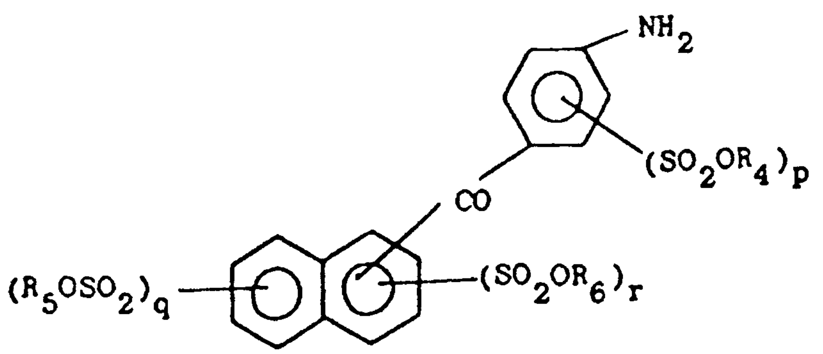

(IIb)

ist, in der ein jeder der Substituenten $R_4$, $R_5$ und $R_6$, die gleich oder verschieden sein können, H oder M darstellt und M die Bedeutung wie im Anspruch 8 hat.

**10.** Phosphoreszenzfähiger Stoff gemäß Anspruch 8 oder 9, wobei die Gesamtheit von p + q + r 2 ist.

**11.** Phosphoreszenzfähiger Stoff gemäß Anspruch 8 oder 9, wobei die Gesamtheit von p + q + r 3 ist.

**12.** Phosphoreszenzfähiger Stoff gemäß Anspruch 8 oder 9, wobei M einem Alkalimetall-Kation entspricht.

**13.** Phosphoreszenzfähiger Stoff gemäß einem jeden der vorangehenden Ansprüche, enthaltend einen weiteren phosporeszierenden Aktivator, der Carbazolsulfonsäure oder ein Salz davon ist.

**14.** Phosphoreszenzfähiger Stoff gemäß Anspruch 13, wobei der phosphoreszierende Aktivator in der Definition von Anspruch 1 und die Carbazolsulfonsäure in einem Gewichtsverhältnis im Bereich von 0,2 bis 1,0, bezogen auf die Natriumsalze beider Aktivatoren, vorhanden sind.

**15.** Phosphoreszenzfähiger Stoff gemäß einem jeden der vorangehenden Ansprüche, wobei die Matrix ein Harz umfaßt, das ein Kondensationsprodukt aus Formaldehyd mit einer Amingruppe enthaltenden Verbindung ist.

**16.** Phosphoreszenzfähiger Stoff gemäß Anspruch 15, wobei die Amingruppen enthaltende Verbindung Harnstoff oder Melamin ist.

**17.** Phosphoreszenzfähiger Stoff gemäß Anspruch 15 oder 16, enthaltend insgesamt von 0,25 bis 10 Gew.-% an dem im Anspruch 1 definierten phosphoreszierenden Aktivator, bezogen auf das Gesamtgewicht von Harz und Aktivator.

**18.** Druckfarbe, enthaltend die Suspension eines organischen phosphoreszenzfähigen Stoffs gemäß einem jeden der vorangehenden Ansprüche in einem geeigneten Medium.

**19.** Vorläufer von einem phosphoreszenzfähigen Stoff, umfassend eine wässerige Lösung, in der als gelöste Stoffe wenigstens ein phosphoreszierender Aktivator aus der Gruppe der in einem der Ansprüche 1 bis 12 definierten, wasserlöslichen Verbindungen und weiterhin ein lösliches Vorkondensat, das durch die Umsetzung zwischen zwei oder mehr Komponenten erhalten wurde und bei einer weiteren Reaktion in Abwesenheit von dem phosphoreszierenden Aktivator ein unlösliches Kondensationsprodukt, das im wesentlichen kein ultraviolettes Licht bei irgendeiner Wellenläge, bei der der phosphoreszierende Aktivator ultraviolettes Licht absorbiert, aufnimmt, bilden würde und das bei einer weiteren Umsetzung in Gegenwart des phosporeszierenden Aktivators ein phosphoreszierendes unlösliches Kondensationsprodukt bildet, enthalten sind.

**20.** Phosphoreszenzfähiger Vorläufer gemäß Anspruch 20, wobei das Gewichtsverhältnis des im Anspruch 1 definierten Aktivators zu der Carbazolsulfonsäure m Bereich von 0,2 bis 1,0, bezogen auf die Natriumsalze der beiden Aktivatoren beträgt.

**21.** Phosphoreszenzfähiger Vorläufer gemäß Anspruch 20, wobei das Gewichtsverhältnis des im Anspruch 1 definierten Aktivators zu der Carbazolsulfonsäure im Bereich von 0,2 bis 1,0, bezogen auf die Natriumsalze der beiden Aktivatoren beträgt.

**22.** Druckfarbe, bestehend im wesentlichen aus einem phosphoreszenzfähigen Vorläufer gemäß einem der Ansprüche 19 bis 21.

**23.** Papierüberzugsmasse, enthaltend einen phosphoreszenzfähigen Vorläufer gemäß einem der Ansprüche 19 bis 21 in Mischung mit einem oder mehreren Füllstoffen und/oder Bindern und/oder Pigmenten und/oder Stabilisatoren und/oder anderen Zusatzstoffen.

## Revendications

**1.** Phosphor organique comprenant des molécules d'activateur de phosphorescence dans une matrice de résine de condensation, qui n'absorbe pas elle-même le rayonnement ultraviolet ou visible aux fréquences d'excitation et d'émission de l'activateur de phosphorescence, lequel activateur de phosphorescence utilise des transitions singulet-triplet et est un composé de formule générale :

$(A)_m$ - Ar - CO - X (I)

dans laquelle Ar représente un groupe aromatique polycyclique à noyaux condensés;

m vaut zéro ou est un nombre entier valant de 1 jusqu'au nombre maximal de positions disponibles pour une substitution sur les noyaux Ar;

X représente un groupe alkyle ou un groupe de formule $(A)_m$-Ar-ou $(B)_n$ $\phi$- dans laquelle $\phi$ représente un groupe phényle et n vaut zéro ou est un nombre entier valant de 1 à 5, et A et B représentent des substituants placés sur les noyaux;

ou l'un de ses sels d'addition d'acide lorsque ledit composé est basique.

**2.** Phosphor conforme à la revendication 1, dans lequel Ar est choisi parmi les groupes naphtyle, anthryle, phénanthryle acénaphtyle et acénaphtényle.

**3.** Phosphor conforme à la revendication 1 ou 2 dans lequel A et B représentent des substituants placés sur les noyaux, choisis parmi les groupes alkyle, cycloalkyle, phényle, alcoxy, cycloalcoxy, phénoxy. hydroxy, amino primaire, hydrazino, amido, carboxyle, formyle, sulfo, halogéno, cyano, azido et phényl-azo, et leurs dérivés substituants.

4. Phosphor conforme à la revendication 1, qui contient, en tant qu'activateur de phosphorescence, du 2-(4-amino-benzoyl)naphtalène.

5. Phosphor conforme à la revendication 1, qui contient, en tant qu'activateur de phosphorescence, du 3-(4-amino-benzoyl) phénanthrène.

6. Phosphor conforme à la revendication 1, qui contient, en tant qu'activateur de phosphorescence, du 2-(napht-2-oyl)naphtalène.

7. Phosphor conforme à la revendication 1, qui contient, en tant qu'activateur de phosphorescence, du 3-(4-aminobenzoyl) acénaphtylène.

8. Phosphor conforme à la revendication 1, dans lequel l'activateur de phosphorescence est un composé, ou un mélange de composés, de formule :

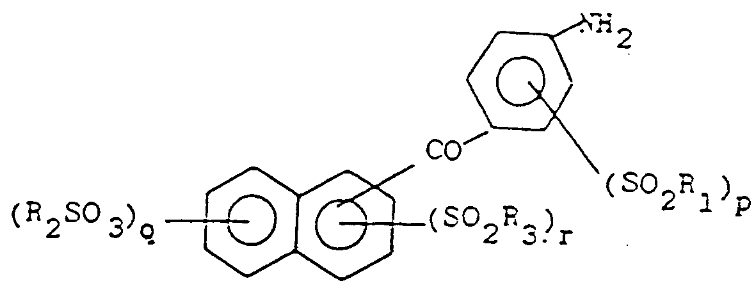

II

dans laquelle :

$R_1$, $R_2$ et $R_3$, qui peuvent être identiques ou différents, représentent chacun -OH, -OM où M représente un cation monovalent ou 1/m d'un cation de valence m, un atome d'halogène, ou -OR où R représente un radical alkyle, aryle ou cycloalkyle, substitué ou non,

p représente zéro ou un nombre entier valant de 1 à 4,

q représente zéro ou un nombre entier valant de 1 à 4, et

r représente zéro ou un nombre entier valant de 1 à 3,

la somme p + q + r valant au moins 1.

9. Phosphor conforme à la revendication 8, dans lequel l'activateur de phosphorescence est un composé, ou un mélange de composés, de formule :

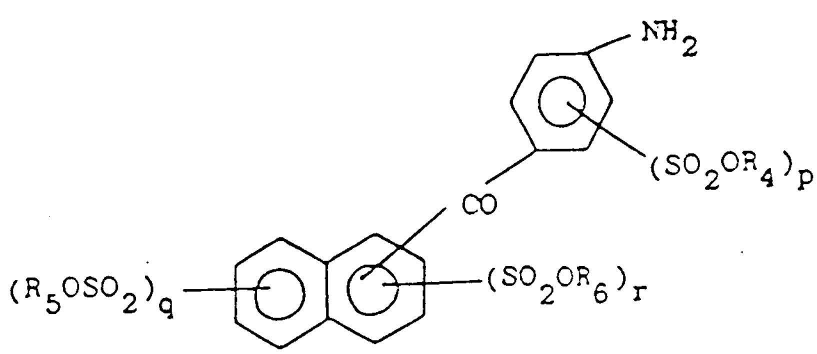

(IIb)

dans laquelle $R_4$, $R_5$ et $R_6$, qui peuvent être identiques ou différents, représentent chacun H ou M, lequel M est tel que défini dans la revendication 8.

10. Phosphor conforme à la revendication 8 ou 9, dans lequel la somme p + q + r vaut 2.

**11.** Phosphor conforme à la revendication 8 ou 9, dans lequel la somme p + q + r vaut 3.

**12.** Phosphor conforme à la revendication 8 ou 9, dans lequel M représente un cation de métal alcalin.

**13.** Phosphor conforme à l'une quelconque des revendications précédentes, qui contient un activateur supplémentaire de phosphorescence qui est un acide carbazole-sulfonique ou un sel d'un tel acide.

**14.** Phosphor conforme à la revendication 13, dans lequel l'activateur de phosphorescence défini dans la revendication 1 et l'acide carbazole-sulfonique se trouvent en un rapport pondéral situé dans l'intervalle allant de 0,2 à 1,0, si on considère les sels de sodium des deux activateurs.

**15.** Phosphor conforme à l'une quelconque des revendications précédentes, dans lequel la matrice comprend une résine qui est un produit de condensation du formaldéhyde avec un composé contenant un groupe amino.

**16.** Phosphor conforme à la revendication 15, dans lequel le composé contenant un groupe amino est de l'urée ou de la mélamine.

**17.** Phosphor conforme à la revendication 15 ou 16, qui contient au total de 0,25% à 10% en poids d'un activateur de phosphorescence tel que défini dans la revendication 1, par rapport au poids total de la résine et de l'activateur.

**18.** Encre d'imprimerie comprenant une suspension d'un phosphor organique tel que revendiqué dans l'une quelconque des revendications précédentes, dans un milieu convenable.

**19.** Précurseur d'un phosphor organique, qui comprend une solution aqueuse comprenant, en tant que soluté, au moins un activateur de phosphorescence qui est un composé défini dans l'une quelconque des revendications 1 à 12, lequel est hydrosoluble, et comprenant en outre, en tant que soluté, un précondensat soluble obtenu par réaction entre deux composants ou plus, le précondensat soluble étant capable de réagir ultérieurement, en l'absence d'activateur de phosphorescence, pour donner un produit de condensation insoluble qui n'absorbe pratiquement pas le rayonnement ultraviolet à toute longueur d'onde à laquelle l'activateur de phosphorescence absorbe pratiquement le rayonnement ultraviolet, et étant capable de réagir ultérieurement, en présence d'un activateur de phosphorescence, pour donner un produit de condensation insoluble phosphorescent.

**20.** Précurseur de phosphor conforme à la revendication 19, qui contient, en tant qu'activateur supplémentaire de phosphorescence, un acide carbazole-sulfonique ou un sel d'un tel acide.

**21.** Précurseur de phosphor conforme à la revendication 20, dans lequel le rapport pondéral de l'activateur défini dans la revendication 1 à l'acide carbazole-sulfonique se situe dans l'intervalle allant de 0,2 à 1,0, si on considère les sels de sodium des deux activateurs.

**22.** Encre d'imprimerie contenant principalement un précurseur de phosphor conforme à l'une quelconque des revendications 19 à 21.

**23.** Composition de couchage de papier, contenant un précurseur de phosphor conforme à l'une quelconque des revendications 19 à 22, en mélange ou conjointement avec un ou plusieurs liants et/ou charges et/ou pigments et/ou stabilisants et/ou autres adjuvants.